# EUROPEAN PATENT APPLICATION

(11) **EP 4 321 609 A1**
(43) Date of publication of application: **14.02.2024**
(21) Application number: 22784890.0
(22) Date of filing: 04.04.2022
(51) Int. Cl.: C12N 5/0775, C12N 5/00

(54) **CELL CULTURE COMPOSITION COMPRISING CHLORELLA EXTRACT**

(30) Priority: 05.04.2021 KR 20210044315
(71) Applicant: Sea With, Inc, Daegu 42988 (KR)
(72) Inventor: CHO, Sung Chun, Incheon 21986 (KR); KIM, Kwan Hyeong, Incheon 21611 (KR); LEE, Tae Byung, Ansan-si, Gyeonggi-do 15329 (KR); JUNG, Chi Sung, Ansan-si, Gyeonggi-do 15320 (KR); KEUM, Joon Ho, Dalseong-gun, Daegu 43019 (KR); LEE, Hee Jae, Dalseong-gun, Daegu 43019 (KR)
(74) Representative: PATERIS Patentanwälte PartmbB
(86) International application number: PCT/KR2022/004805
(87) International publication number: WO 2022/215980

(57) **Abstract**

The present disclosure relates to a cell culture composition including a chlorella extract.

According to the cell culture medium composition including the chlorella extract according to an aspect, the medium including the chlorella extract can improve the cell proliferation ability of bovine myogenic stem cells. Thus, the chlorella extract can be used as a substitute for fetal bovine serum.

## Description

### Technical Field

The present disclosure relates to a composition including a chlorella extract for cell culture.

### Background Art

Cell culture is the most basic and important experimental technique today for research in the medical field as well as in molecular biology and immunology. In animal cell culture methods that have been used so far, serum must be supplied to a cell culture medium for growth, and the most widely used serum is fetal bovine serum (FBS). Also, even when culturing adult stem cells and inducing differentiation of adult stem cells, various types of differentiation-inducing factors have been added to an FBS-containing medium.

However, due to the use of animal-derived protein sources including FBS in the cell culture process using the medium, it is difficult to exclude stability problems such as cross-species contamination. Also, in the manufacturing process of FBS, a bovine fetus is harvested from the uterus of a pregnant mother cow to separate serum, and in this regard, ethical problems inevitably arise. The international communities including the U.S. FDA and the European Medicines Agency (EMEA), have advised against the use of FBS and the development of alternatives to FBS. Furthermore, the controversy over the risk of transmission of FBS-caused bovine spongiform encephalopathy is still unresolved.

As a result of achieving active research on cell culture media to replace FBS, various serum-free media have been developed. However, in the case of serum-free media developed so far, applicable cell lines are very limited, the adaptation process of cell lines for culture is difficult, and the cell division and growth speed are slow. Furthermore, since additional addition of various hormones and growth factors essential for cell culture is required, there are disadvantages causing inconvenience to users.

Therefore, the development of a culture composition that can replace FBS is continuously required.

### Disclosure

### Technical Problem

An aspect provides a medium composition for cell culture, including a chlorella extract.

Another aspect provides a medium additive for cell culture, including a chlorella extract.

Another aspect provides a method for culturing a cell by using the medium composition for cell culture.

### Technical Solution

An aspect is to provide a medium composition for cell culture, including a chlorella extract.

The term "cell culture" as used in the present specification refers to a process of artificially growing living cells *in vitro* under controlled conditions. In addition, cells may be grown and proliferated by spreading a suspension, in which a portion of an individual's tissue is aseptically removed and intercellular linking materials are dissolved and liberated with an enzyme, on a flat bottom of a bottle or a culture dish such as a Petri dish.

The cell culture may include culturing cells to prepare cell cultured meat. Therefore, the composition may be a medium composition for preparing cultured meat. The medium composition of the present disclosure may include a chlorella extract, which is an edible material, instead of commonly used fetal bovine serum. The chlorella extract is a material rich in nutrients such as proteins and amino acids, and can eliminate safety issues. By having a low production cost compared to fetal bovine serum, excellent productivity of the chlorella extract is expected.

The term "cultured meat" as used in the present specification refers to edible meat obtained by harvesting animal cells and proliferating the cells by cell engineering technology, and is regarded as a field of cellular agriculture in which meat is obtained without undergoing livestock breeding. In Korean, such meat is called cultured meat, alternative meat, or artificial meat. Meanwhile, in English, it is called in vitro meat in the sense that meat is grown in a test tube, artificial meat in the sense that not native cells, but stem cells are used by humans for synthesis, clean meat in the sense that meat is produced in a clear production facility, not a traditional breeding facility, or lab-grown meat in the sense that meat is produced in a laboratory.

The term "culture medium" as used in the present specification refers to a medium capable of supporting growth, survival, and differentiation of cells or stem cells *in vitro,* and includes all conventional media suitable for culturing and differentiating cells or stem cells used in the corresponding field. Depending on types of cells, types of media and culture conditions can be selected at the technical level in the corresponding field. A medium used for culture is a cell culture minimum medium (CCMM), and generally contains a carbon source, a nitrogen source, and trace element ingredients. The CCMM may be, for example, at least one selected from the group consisting of Dulbecco's Modified Eagle's Medium (DMEM), Minimal Essential Medium (MEM), Basal Medium Eagle (BME), RPMI 1640, F-10, F-12, DMEM/F12, α-Minimal Essential Medium (α-MEM), Glasgow's Minimal Essential Medium (G-MEM), Iscove's Modified Dulbecco's Medium (IMDM), MacCoy's 5A medium, AmnioMax complete medium, AminoMax ±medium, Endothelial Basal Medium (EBM), Chang's Medium, MesenCult-XF, Dulbecco's Modified Eagle's Medium high glucose (DMEM/HG), and MCDB+Dulbecco's Modified Eagle's Medium low glucose (MCDB+DMEM/LG). In addition, the medium may contain antibiotics, such as penicillin, streptomycin, gentamicin, or a mixture or two or more thereof. Therefore, the medium composition for cell culture of the present disclosure may include a chlorella extract added to the CCMM.

The term "chlorella" as used in the present specification refers to a green alga belonging to the phylum of green algae. Chlorella is a planktonic unicellular alga that is bright green and consists of microscopic spherical or ovoid cells with a diameter of 10 µm or less. It has one nucleus and one chloroplast and is non-motile due to a lack of flagella, and each individual grows separately by floating in water. Dried chlorella contains, per 100 g, 40 g to 50 g of protein, 10 g to 25 g of carbohydrate, and 10 g to 30 g of lipid, and is considered as a good source of microbial protein as being abundant in essential amino acids such as lysine and methionine.

The chlorella may be any species belonging to the genus *Chlorella* and being available to those skilled in the art. In detail, the chlorella may be at least one selected from the group consisting of Chlorella vulgaris, Chlorella pyrenoidosa, Chlorella regularis, Chlorella saccharophila, Chlorella sorokiniana, Chlorella capsulata, and the like, but is not limited thereto.

The term "extract" as used herein refers to a product, such as a liquid component, which is obtained by immersing a target material in various solvents and then extracting at room temperature or elevated temperature for a certain period of time, a solid component, which is obtained by removing the solvent from the liquid component, and the like. Furthermore, in addition to the product, the term can be comprehensively interpreted as including the extract itself and extracts of all formulations that can be formed using the extract, such as a dilution of the product, a concentrate thereof, a crude purified product thereof, a purified product thereof, or a mixture thereof. In addition, the extract may include a fraction obtained therefrom.

The extract may be extracted from natural, crossbred, or mutant chlorella, and may also be extracted from a chlorella tissue culture.

Here, an extraction solvent for extraction of the extract may be a polar protic or polar aprotic solvent and a nonpolar solvent. The polar protic solvent may be water, methanol, ethanol, propanol, isopropanol, or butanol. The polar aprotic solvent may be dichloromethane, tetrahydrofuran, ethylacetate, acetonitrile, dimethylformamide, dimethylsulfoxide, acetone, 2-butanone, or hexamethylphosphoramide. The nonpolar solvent may be pentane, hexane, chloroform, or diethylether. The nonpolar solvent excludes benzene. The solvent may be C1-C6 alcohol, C3-C10 ester, such as C3-C10 acetate, C3-C10 ketone, unsubstituted or halogenated C1-C6 hydrocarbon, C2-C10 cyclic ether, a mixture thereof, or a mixture of water and one or more of the aforementioned solvent. The solvent may be ethanol, propanol, acetonitrile, ethylacetate, acetone, 2-butanone, chloroform, dichloromethane, hexane, a mixture thereof, or a mixture of water and one or more of the aforementioned solvent. The hydrocarbon may be alkane, alkene, or alkyne.

The extract may be extracted by using one or more solvents selected from the group consisting of: water; C1 to C4 low alcohol, such as methanol, ethanol, propanol, and butanol; polyhydric alcohol, such as glycerine, butylene glycol, or propylene glycol; and hydrocarbon-based solvent, such as methyl acetate, ethyl acetate, acetone, benzene, hexane, diethyl ether, or dichloromethane. In detail, the extract may be extracted by using water as a solvent.

As the chlorella extract, an extract obtained by method in the art may be used. For example, an extraction method such as hot water extraction, cold water extraction, high temperature extraction under reduced pressure, boiling water extraction, room temperature extraction, fraction extraction, reflux cooling extraction, solvent extraction, steam distillation, ultrasonification extraction, elusion, press, and the like may be used. In addition, as necessary, a commercially available chlorella extract may be used as the chlorella extract.

The term "fraction" as used in the present specification refers to a product obtained by performing fractionation to separate a specific component or a specific component group from a mixture containing various components.

A fractionation method for obtaining the fraction is not particularly limited, and may be performed according to a method commonly used in the art. Non-limiting examples of the fractionation method include fractionation performed by treating various solvents, ultrafiltration fractionation performed by passing an ultrafiltration film having a constant molecular weight cut-off value, chromatography fractionation that performs various types of chromatography (designed for separation according to size, charge, hydrophobicity, or affinity), and a combination thereof.

Types of a fractionation solvent used to obtain the fraction are not particularly limited, and any solvent known in the art may be used. Non-limiting examples of the fractionation solvent include: a polar solvent, such as water, C1-C4 alcohol, etc.; a nonpolar solvent, such as hexane, ethyl acetate, chloroform, dichloromethane, etc.; or mixed solvents thereof. The fractionation solvent may be used alone or in combination of one or more solvents, but is not limited thereto.

The chlorella extract may be included in the medium composition for cell culture at a concentration of 25 g/L or less, specifically, 23 g/L or less. For example the concentration may be 0.001 g/L to 25 g/L, 0.005 g/L to 25 g/L, 0.01 g/L to 25 g/L, 0.05 g/L to 25 g/L, 0.1 g/L to 25 g/L, 0.2 g/L to 25 g/L, 0.4 g/L to 25 g/L, 0.8 g/L to 25 g/L, 1 g/L to 25 g/L, 2 g/L to 25 g/L, 3 g/L to 25 g/L, 4 g/L to 25 g/L, 6 g/L to 25 g/L, 8 g/L to 25 g/L, 10 g/L to 25 g/L, 15 g/L to 25 g/L, 20 g/L to 25 g/L, 0.001 g/L to 23 g/L, 0.005 g/L to 23 g/L, 0.01 g/L to 23 g/L, 0.05 g/L to 23 g/L, 0.1 g/L to 23 g/L, 0.2 g/L to 23 g/L, 0.4 g/L to 23 g/L, 0.8 g/L to 23 g/L, 1 g/L to 23 g/L, 2 g/L to 23 g/L, 3 g/L to 23 g/L, 4 g/L to 23 g/L, 6 g/L to 23 g/L, 8 g/L to 23 g/L, 10 g/L to 23 g/L, 15 g/L to 23 g/L, 20 g/L to 23 g/L, 0.001 g/L to 22 g/L, 0.005 g/L to 22 g/L, 0.01 g/L to 22 g/L, 0.05 g/L to 22 g/L, 0.1 g/L to 22 g/L, 0.2 g/L to 22 g/L, 0.4 g/L to 22 g/L, 0.8 g/L to 22 g/L, 1 g/L to 22 g/L, 2 g/L to 22 g/L, 3 g/L to 22 g/L, 4 g/L to 22 g/L, 6 g/L to 22 g/L, 8 g/L to 22 g/L, 10 g/L to 22 g/L, 15 g/L to 22 g/L, 20 g/L to 22 g/L, 0.001 g/L to 20 g/L, 0.005 g/L to 20 g/L, 0.01 g/L to 20 g/L, 0.05 g/L to 20 g/L, 0.1 g/L to 20 g/L, 0.2 g/L to 20 g/L, 0.4 g/L to 20 g/L, 0.8 g/L to 20 g/L, 1 g/L to 20 g/L, 2 g/L to 20 g/L, 3 g/L to 20 g/L, 4 g/L to 20 g/L, 6 g/L to 20 g/L, 8 g/L to 20 g/L, 10 g/L to 20 g/L, 15 g/L to 20 g/L, 0.001 g/L to 15 g/L, 0.005 g/L to 15 g/L, 0.01 g/L to 15 g/L, 0.05 g/L to 15 g/L, 0.1 g/L to 15 g/L, 0.2 g/L to 15 g/L, 0.4 g/L to 15 g/L, 0.8 g/L to 15 g/L, 1 g/L to 15 g/L, 2 g/L to 15 g/L, 3 g/L to 15 g/L, 4 g/L to 15 g/L, 6 g/L to 15 g/L, 8 g/L to 15 g/L, 10 g/L to 15 g/L, 0.001 g/L to 12 g/L, 0.005 g/L to 12 g/L, 0.01 g/L to 12 g/L, 0.05 g/L to 12 g/L, 0.1 g/L to 12 g/L, 0.2 g/L to 12 g/L, 0.4 g/L to 12 g/L, 0.8 g/L to 12 g/L, 1 g/L to 12 g/L, 2 g/L to 12 g/L, 3 g/L to 12 g/L, 4 g/L to 12 g/L, 6 g/L to 12 g/L, 8 g/L to 12 g/L, 10 g/L to 12 g/L, 0.001 g/L to 11 g/L, 0.005 g/L to 11 g/L, 0.01 g/L to 11 g/L, 0.05 g/L to 11 g/L, 0.1 g/L to 11 g/L, 0.2 g/L to 11 g/L, 0.4 g/L to 11 g/L, 0.8 g/L to 11 g/L, 1 g/L to 11 g/L, 2 g/L to 11 g/L, 3 g/L to 11 g/L, 4 g/L to 11 g/L, 6 g/L to 11 g/L, 8 g/L to 11 g/L, 10 g/L to 11 g/L, 0.001 g/L to 10 g/L, 0.005 g/L to 10 g/L, 0.01 g/L to 10 g/L, 0.05 g/L to 10 g/L, 0.1 g/L to 10 g/L, 0.2 g/L to 10 g/L, 0.4 g/L to 10 g/L, 0.8 g/L to 10 g/L, 1 g/L to 10 g/L, 2 g/L to 10 g/L, 3 g/L to 10 g/L, 4 g/L to 10 g/L, 6 g/L to 10 g/L, 8 g/L to 10 g/L, 0.001 g/L to 8 g/L, 0.005 g/L to 8 g/L, 0.01 g/L to 8 g/L, 0.05 g/L to 8 g/L, 0.1 g/L to 8 g/L, 0.2 g/L to 8 g/L, 0.4 g/L to 8 g/L, 0.8 g/L to 8 g/L, 1 g/L to 8 g/L, 2 g/L to 8 g/L, 3 g/L to 8 g/L, 4 g/L to 8 g/L, 6 g/L to 8 g/L, 0.001 g/L to 5 g/L, 0.005 g/L to 5 g/L, 0.01 g/L to 5 g/L, 0.05 g/L to 5 g/L, 0.1 g/L to 5 g/L, 0.2 g/L to 5 g/L, 0.4 g/L to 5 g/L, 0.8 g/L to 5 g/L, 1 g/L to 5 g/L, 2 g/L to 5 g/L, 3 g/L to 5 g/L, 4 g/L to 5 g/L, 0.001 g/L to 4 g/L, 0.005 g/L to 4 g/L, 0.01 g/L to 4 g/L, 0.05 g/L to 4 g/L, 0.1 g/L to 4 g/L, 0.2 g/L to 4 g/L, 0.4 g/L to 4 g/L, 0.8 g/L to 4 g/L, 1 g/L to 4 g/L, 2 g/L to 4 g/L, or 3 g/L to 4 g/L, and specifically, may be 0.8 g/L to 4 g/L.

The cells may be somatic cells, embryonic cells, reproductive cells, stem cells, cancer cells, cell lines, *in vitro* cells, graft cells, primary cells (*in vitro* and ex *vivo*), *in vivo* cells, cells of eukaryotic organisms, or mammalian cells including humans. For example, the cells may be selected from the group consisting of cancer cells, stem cells, vascular endothelial cells, leukocytes, immune cells, epithelial cells, reproductive cells, fibroblasts, muscle cells, bone marrow cells, epidermal cells, osteoblasts, and neurons. In addition, the eukaryotic organism may be an insect, an amphibian, a bird (including chicken, turkey, duck, etc.), or a mammal (e.g., primate, such as human and monkey, dog, pig, cow, sheep, goat, mouse, rat, etc.).

The cells may be separated from an individual or an eukaryotic organism.

The cells may be undifferentiated cells, cells being in differentiation, or differentiated cells, and for example, may be stem cells.

The term "stem cell" refers to a cell having potency and self-renewal ability. The stem cells are classified into pluripotency, multipotency, or unipotency according to differentiation ability. The stem cells may include one or more selected from the group consisting of embryonic stem cells (ESCs) (inner cells of preimplantation embryo), adult stem cells (undifferentiated cells present in each tissue and organ), and dedifferentiation stem cells (cells in which dedifferentiation is induced by inserting genes and/or proteins into somatic cell, or induced pluripotent stem cells (iPSCs)).

Types or origins of the stem cells are not limited as long as the cells have the differentiation and self-renewal ability. The stem cells may be derived from, for example, mammals, humans, monkeys, pigs, horses, cows, sheep, dogs, cats, mice, or rabbits. The stem cells may be derived from separated umbilical cord, placenta, adipose, bone marrow, muscle, cord blood, or amniotic fluid. The term "separated" refers to presence in an environment different from that of naturally occurring cell or tissue. Separation of umbilical cord, placenta, adipose, bone marrow, muscle, cord blood, or amniotic fluid and obtaining stem cells therefrom may be performed by conventional anatomical methods and known methods in the art.

The cells may be muscle cells or myogenic stem cells, and specifically may be derived from livestock such as chicken, bovine, and porcine, etc., and more specifically may be derived from bovine or porcine muscle cells or bovine or porcine myogenic stem cells. In addition, the myogenic stem cells may be, for example, derived from at least one selected from the group consisting of chuck, sirloin, striploin, tenderloin, rump, bottom round, front leg, brisket, rib, shank/shin, and the like.

The term "myogenic stem cell" as used in the present specification refers to cells having characteristics of myogenic stem cells including all of proliferation without transformation, infinite proliferation, self-renewal ability, and ability to differentiate into muscles. The myogenic stem cell may include any cell without limitation as long as having self-renewal ability or infinite proliferation ability and muscle differentiation ability. The self-renewal ability and muscle differentiation ability may be confirmed by using markers. The myogenic stem cells may be used interchangeably with myogenic satellite cells (SCs).

In an embodiment, it is confirmed that the medium composition including the chlorella extract does not exhibit cell proliferation efficacy for fibroblasts, but exhibits excellent cell proliferation efficacy for myogenic stem cells.

The composition may further include serum. The serum may be at least one selected from the group consisting of fetal bovine serum (FBS), bovine calf serum (BCS), human serum, and horse serum (HS), and specifically may be FBS.

The amount of the serum may be 0.5 parts by volume to 15 parts by volume based on 100 parts by volume of the total amount of the composition for cell culture. For example, the amount of the serum contained may be, based on 100 parts by volume, 0.5 parts by volume to 15 parts by volume, 0.5 parts by volume to 13 parts by volume, 0.5 parts by volume to 11 parts by volume, 0.5 parts by volume to 10 parts by volume, 0.5 parts by volume to 9 parts by volume, 0.5 parts by volume to 8parts by volume, 0.5 parts by volume to 7 parts by volume, 0.5 parts by volume to 6 parts by volume, 0.5 parts by volume to 5 parts by volume, 1 parts by volume to 15 parts by volume, 1 parts by volume to 13 parts by volume, 1 parts by volume to 11 parts by volume, 1 parts by volume to 10 parts by volume, 1 parts by volume to 9 parts by volume, 1 parts by volume to 8 parts by volume, 1 parts by volume to 7 parts by volume, 1 parts by volume to 6 parts by volume, 1 parts by volume to 5 parts by volume, 2 parts by volume to 15 parts by volume, 2 parts by volume to 13 parts by volume, 2 parts by volume to 11 parts by volume, 2 parts by volume to 10 parts by volume, 2 parts by volume to 9 parts by volume, 2 parts by volume to 8 parts by volume, 2 parts by volume to 7 parts by volume, 2 parts by volume to 6 parts by volume, 2 parts by volume to 5 parts by volume, 4 parts by volume to 15 parts by volume, 4 parts by volume to 13 parts by volume, 4 parts by volume to 11 parts by volume, 4 parts by volume to 10 parts by volume, 4 parts by volume to 9 parts by volume, 4 parts by volume to 8 parts by volume, 4 parts by volume to 7 parts by volume, 4 parts by volume to 6 parts by volume, 4 parts by volume to 5 parts by volume, 5 parts by volume to 15 parts by volume, 5 parts by volume to 13 parts by volume, 5 parts by volume to 11 parts by volume, 5 parts by volume to 10 parts by volume, 5 parts by volume to 9 parts by volume, 5 parts by volume to 8 parts by volume, 5 parts by volume to 7 parts by volume, or 5 parts by volume to 6 parts by volume, and for example, 0.5 parts by volume to 11 parts by volume, 1 parts by volume to 11 parts by volume, 2 parts by volume to 11 parts by volume, 4 parts by volume to 11 parts by volume, 1 parts by volume to 10 parts by volume, 2 parts by volume to 10 parts by volume, 4 parts by volume to 10 parts by volume, or 4 parts by volume to 6 parts by volume.

The composition may contain serum and the chlorella extract, and the serum may be contained in a weight ratio range of 1000:1 to 1:1 compared to the chlorella extract (serum:chlorella extract). For example the weight ratio of the serum to the chlorella extract may be in a range of 1000:1 to 1:1, 800:1 to 1:1, 600:1 to 1:1, 500:1 to 1:1, 400:1 to 1:1, 350:1 to 1:1, 300:1 to 1:1, 250:1 to 1:1, 200:1 to 1:1, 150:1 to 1:1, 100:1 to 1:1, 50:1 to 1:1, 40:1 to 1:1, 30:1 to 1:1, 25:1 to 1:1, 20:1 to 1:1, 15:1 to 1:1, 12.5:1 to 1:1, 10:1 to 1:1, 7.5:1 to 1:1, 5:1 to 1:1, or2.5:1 to 1:1.

The composition for cell culture may be capable of subculture of cells.

The term "passage" as used in the present specification refers to replacing a culture container or culturing by dividing cell population in a method for continuously culturing cells, specifically stem cells, in a healthy state for a long period of time. Replacing a culture container once or culturing by dividing cell population once is called passage 1. In the present disclosure, the passage may be used interchangeably with generation.

The culture may refer to growth and proliferation. The term "growth and proliferation" as used in the present specification refers to an increase in the number of cells. The culture may refer to undifferentiated proliferation. The undifferentiated proliferation refers that stem cells proliferate into cells having the same properties as the original cells, i.e., having potency and self-renewal ability, without being differentiated into specific cells. The term "differentiation" refers to a phenomenon in which cells become specialized in each structure or function during growth by division and proliferation, that is, a change of forms or functions of cells, tissues, etc., of living organisms to perform the task given thereto. Measuring or determining the degree of differentiation into a specific cell type can be performed by methods well known in the art. In addition, the differentiation can be confirmed by examining cell morphology using an optical microscope or a confocal microscope or by measuring changes in gene expression using techniques well known in the art, such as polymerase chain reaction (PCR) and gene-expression profile, while measuring changes in cell surface markers (e.g., by staining cells with tissue-specific or cell-lab marker-specific antibodies) and cell morphology using techniques such as flow cytometry or immunocytochemistry.

Another aspect is to provide a medium additive for cell culture, including the chlorella extract. The same contents as described above also apply to the descriptions of the composition.

Since the medium additive can replace all or part of the serum, a medium for cell culture containing the medium additive may have a reduced serum amount compared to a general culture medium (about 10 % serum). In addition, a low-serum culture medium containing the medium additive may exhibit cell growth rates, cell viability, and subculture efficiency that are equivalent to or significantly better than those of a 10 % serum containing-culture medium.

Another aspect is to provide a method for cell culture, including culturing separated cells by using the medium composition for cell culture. The same contents as described above also apply to the descriptions of the method.

Here, the separated cells may be cells separated from livestock, and specifically may be cells separated from livestock such as cows, pigs, or chickens.

The cells may be myogenic stem cells, and specifically may be myogenic stem cells separated from muscle tissue of livestock.

The method for cell culture may be a method for culturing cells to prepare cultured meat, and specifically may be a method for proliferating and culturing myogenic stem cells for preparing cultured meat. Therefore, cultured meat may be prepared by using the cells, e.g., myogenic stem cells, cultured by the method.

The culture form may be a conventional two-dimensional or three-dimensional culture known in the art. However, to implement a tissue similar to an actual biological tissue by intercellular interactions, three-dimensional culture may be preferable. Specific examples of three-dimensional culture include 3D porous scaffolds, scaffold-free platforms using cells themselves or cell sheet technology, methods of arranging cells in microchips, methods using hydrogels, or methods using a bioreactor.

Another aspect is to provide a method for preparing cultured meat, the method including culturing separated cells by using the medium composition for cell culture. The same contents as described above also apply to the descriptions of the method.

Here, the separated cells may be cells separated from livestock, and specifically may be cells separated from livestock such as cows, pigs, or chickens.

The cells may be myogenic stem cells, and specifically may be myogenic stem cells separated from muscle tissue of livestock.

The method may include differentiating the cultured cells into myofibers and/or muscular tissues, and specifically may include differentiating the cultured cells into myocytes and/or myotubes, followed by differentiating the myocytes and/or myotubes into myofibers and/or muscular tissues.

The differentiating may be performed under conditions including one or more selected from the group consisting of a scaffold, a differentiation culture medium, and a physical stimulus.

The culture form at the differentiating may be a conventional two-dimensional or three-dimensional culture known in the art. However, to implement a tissue similar to an actual biological tissue by intercellular interactions, three-dimensional culture may be preferable. Specific examples of three-dimensional culture include 3D porous scaffolds, scaffold-free platforms using cells themselves or cell sheet technology, methods of arranging cells in microchips, methods using hydrogels, or methods using a bioreactor.

### Advantageous Effects

According to a cell culture medium composition including a chlorella extract according to an aspect, a medium including the chlorella extract can improve the cell proliferation ability of myogenic stem cells. Thus, the chlorella extract can be used as a substitute for fetal bovine serum that is generally used in cell culture.

### Description of Drawings

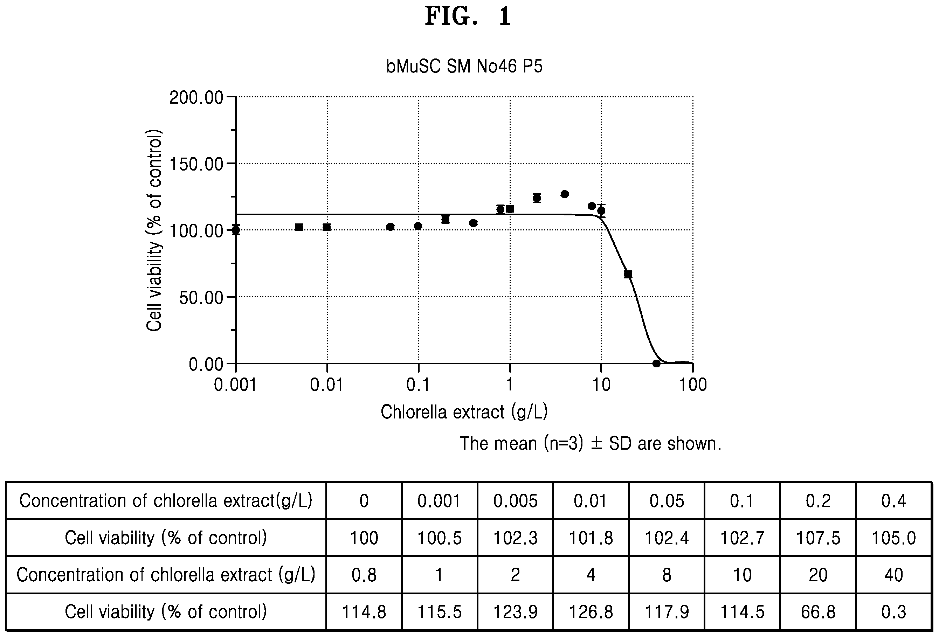

FIG. 1 is a view showing the cytotoxicity and cell proliferation effect of a chlorella extract on bovine shank/shin myogenic stem cells. The results are expressed as mean (N=3) ± standard deviation.

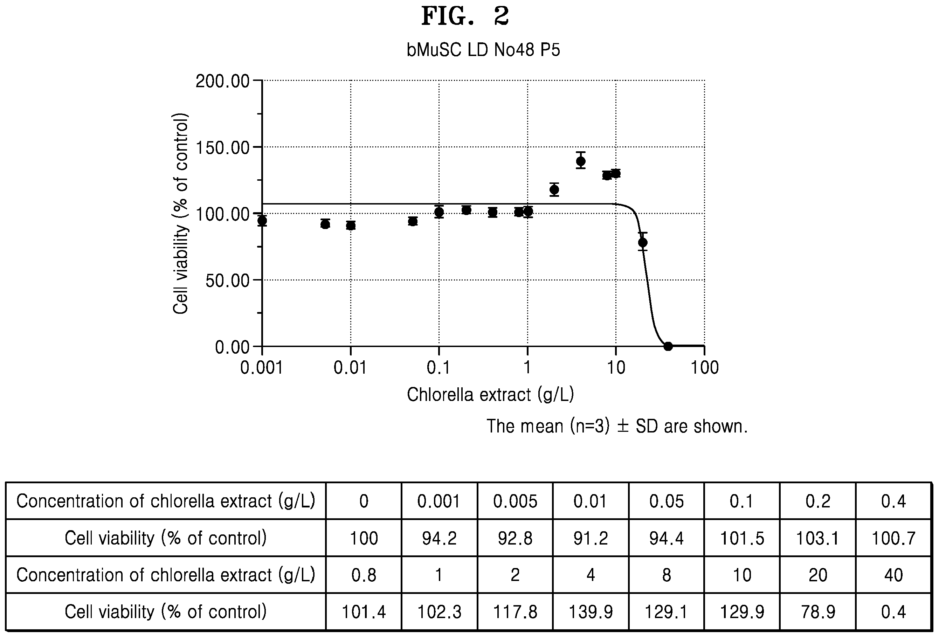

FIG. 2 is a view showing the cytotoxicity and cell proliferation effect of a chlorella extract on bovine sirloin myogenic stem cells. The results are expressed as mean (N=3) ± standard deviation.

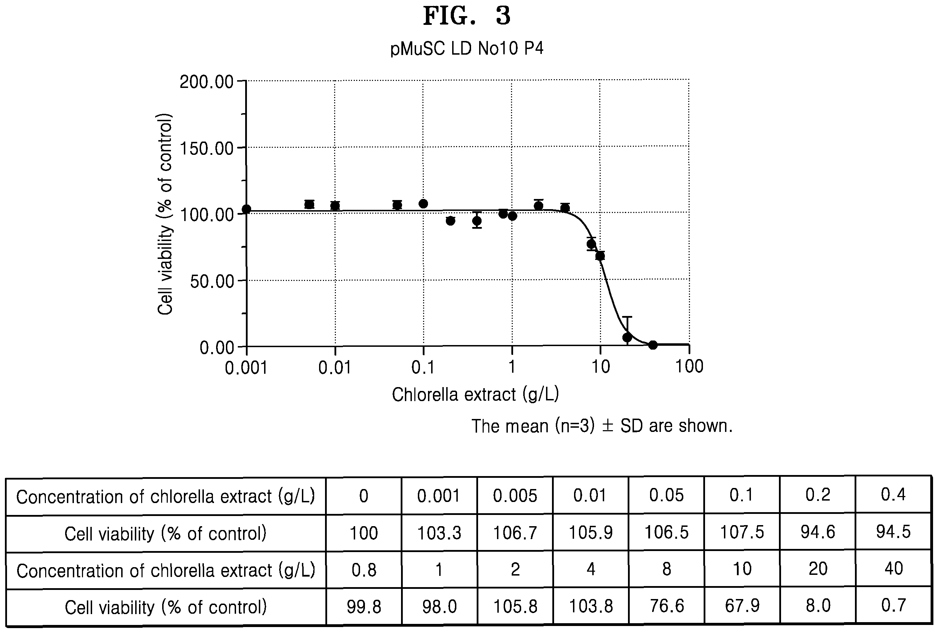

FIG. 3 is a view showing the cytotoxicity and cell proliferation effect of a chlorella extract on porcine sirloin myogenic stem cells. The results are expressed as mean (N=3) ± standard deviation.

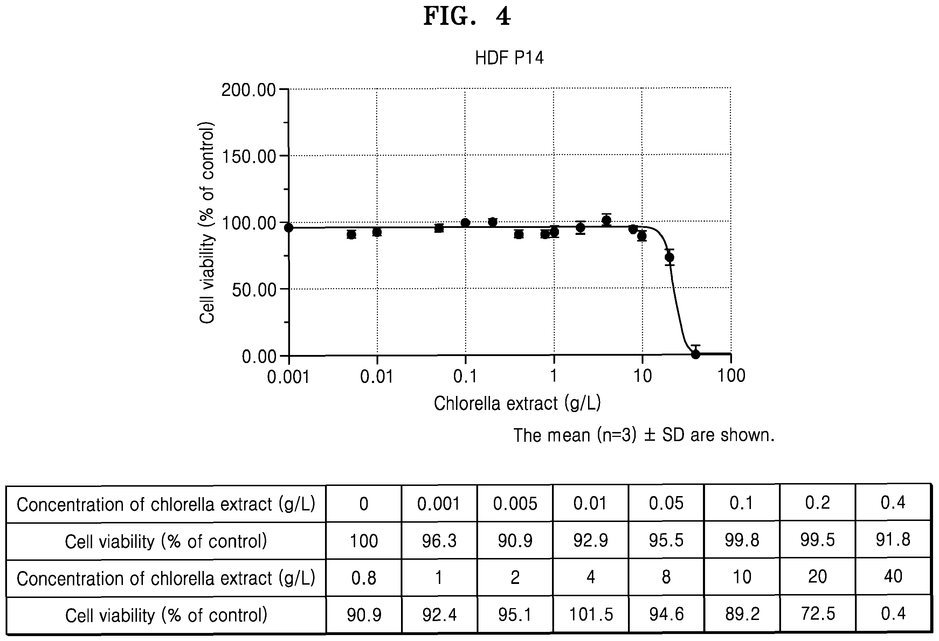

FIG. 4 is a view showing the cytotoxicity and cell proliferation effect of a chlorella extract on human dermal fibroblasts. The results are expressed as mean (N=3) ± standard deviation.

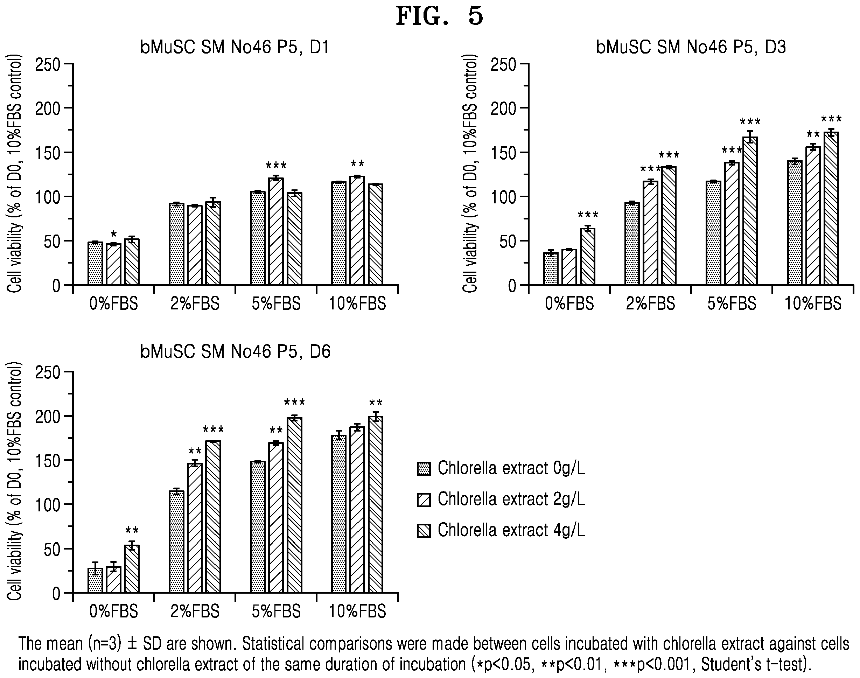

FIG. 5 is a view showing the cell proliferation effect of a culture solution containing FBS at various concentrations and a chlorella extract at various concentrations on bovine shank/shin myogenic stem cells. The results are expressed as mean (N=3) ± standard deviation, and statistical comparison is made between cells cultured without a chlorella extract and cells cultured in a medium containing a chlorella extract, during the same culture period. (* p <0.05, ** p <0.01, *** p <0.001, Student's T test).

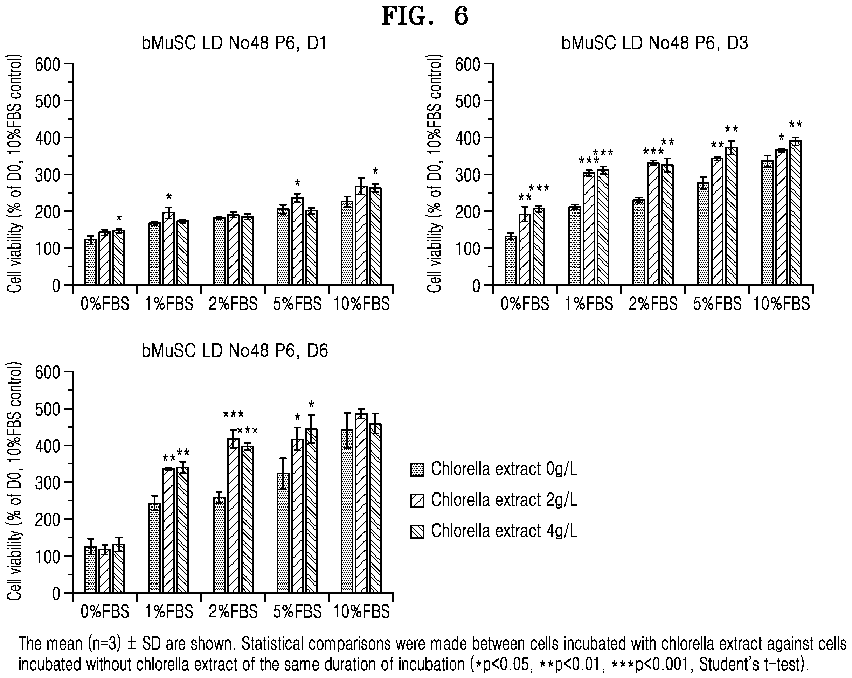

FIG. 6 is a view showing the cell proliferation effect of a culture solution containing FBS at various concentrations and a chlorella extract at various concentrations on bovine sirloin myogenic stem cells. The results are expressed as mean (N=3) ± standard deviation, and statistical comparison is made between cells cultured without a chlorella extract and cells cultured in a medium containing a chlorella extract, during the same culture period. (* p <0.05, ** p <0.01, *** p <0.001, Student's T test).

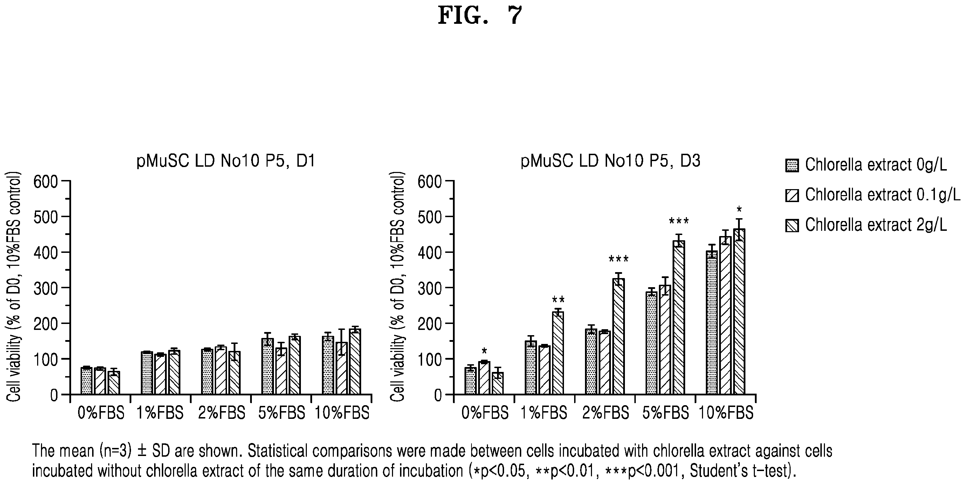

FIG. 7 is a view showing the cell proliferation effect of a culture solution containing FBS at various concentrations and a chlorella extract at various concentrations on porcine sirloin myogenic stem cells. The results are expressed as mean (N=3) ± standard deviation, and statistical comparison is made between cells cultured without a chlorella extract and cells cultured in a medium containing a chlorella extract, during the same culture period. (* p <0.05, ** p <0.01, *** p <0.001, Student's T test).

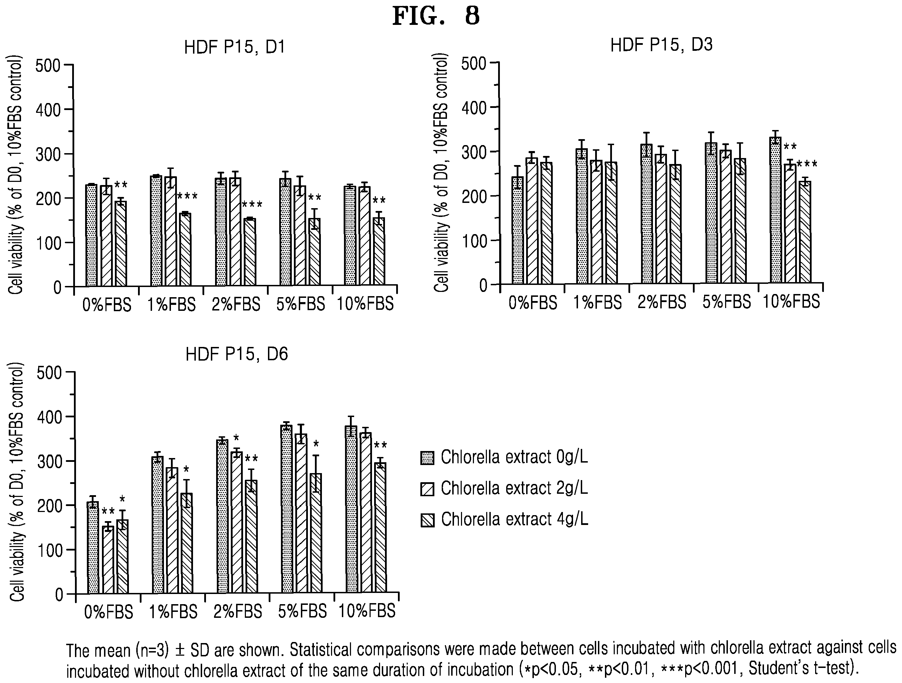

FIG. 8 is a view showing the cell proliferation effect of a culture solution containing FBS at various concentrations and a chlorella extract at various concentrations on human dermal fibroblasts. The results are expressed as mean (N=3) ± standard deviation, and statistical comparison is made between cells cultured without a chlorella extract and cells cultured in a medium containing a chlorella extract, during the same culture period. (* p <0.05, ** p <0.01, *** p <0.001, Student's T test).

### BEST MODE

### Mode for Invention

Hereinafter, the present disclosure will be described in more detail with reference to Examples below. However, these Examples are for illustrative purposes only, and the scope of the present disclosure is not intended to be limited by these Examples.

### Example 1: Obtaining of chlorella extract and cell culturing

A chlorella extract used in this Example was prepared by performing hot water extraction on heterotrophically cultured *Chlorella vulgaris.* In detail, the chlorella raw material cultured heterotrophically was dehydrated through centrifugation, and then dried at 50 °C. The dried chlorella was pulverized by using a grinder. The chlorella powder was dissolved in purified water at a ratio of 1:50 w/v, and hot water extraction was performed thereon by heating at 70 °C for 4 hours. The extract undergone the hot water extraction was filtered and concentrated by using a rotary evaporator under conditions of a temperature of 50 °C and a speed of 50 rpm. Finally, the concentrated hot-water extract was lyophilized and pulverized to obtain a chlorella extract. For culturing of bovine myogenic stem cells (bMuSCs)(shank/shin and sirloin), porcine myogenic stem cells (pMuSCs), and human dermal fibroblasts (HDFs), a Dulbecco's Modified Eagle's medium (DMEM) supplemented with 10 % fetal bovine serum (FBS) and 1 % penicillin-streptomycin (PS) was used. By culturing the cells in a T-25 cell culture flask, necessary cells were proliferated. When the confluency reached 80 % to 90 %, the cells were detached and used for experiments. In addition, the myogenic stem cells of the shank/shin were obtained from semimembranosus (SM) muscles in the shank/shin area, and the myogenic stem cells of the sirloin were obtained from longissimus dorsi (LD) muscles in the sirloin area.

### Example 2: Confirmation of cytotoxicity and proliferative ability of chlorella extract

To confirm the effects (cytotoxicity and proliferative ability) of the chlorella extract on the bovine muscle cells, porcine muscle cells, and human dermal fibroblasts, the following experiments were carried out.

In detail, to confirm the cytotoxicity, a WST-8 assay was carried out, and passage 3 or 5 cells were seeded at a concentration of 5,000 cells per well in a 96-well culture plate (seeded into 3 wells for each condition). The cells were cultured for one day under conditions of a temperature of 37 °C and 5 % CO₂, and the culture medium was replaced with a new culture medium supplemented with 10 % FBS and the chlorella extract at different concentrations (0.001 g/L to 40 g/L). After the cells were cultured for 3 days, each well was treated with 5 µL of WST-8, and then additionally cultured for 3 hours. The metabolic activity of the cells was determined by measuring absorbance at a wavelength of 450 nm by using a microplate spectrophotometer. Wells containing only the culture medium without the cells and wells not treated with the chlorella extract were used as blank control and negative control, respectively. By comparing the OD450 value of the cells treated with the chlorella extract with the OD450 value of the negative control, the relative cell viability was compared. The concentration of the chlorella extract showing the highest viability was named Eₘₐₓ, and the CC₅₀ value at which the cell viability was 50 % of that of the negative control was calculated by using a Quest Graph^{™} IC50 calculator.

As a result, the cell proliferation rate and viability according to the concentration of the chlorella extract are shown in FIGS. 1 to 4.

In detail, the cell proliferation of bMuSCs SM increased when the chlorella extract was included at a concentration of 0.001 g/L to 10 g/L. In particular, when included at a concentration of 4 g/L, the cell proliferation increased by 126.8 % compared to the control, showing the highest increase rate. The concentration of 4 g/L was identified as Eₘₐₓ. In addition, the CC₅₀ value, which is the concentration showing 50 % of cytotoxicity, was calculated to be 22.7 g/L (FIG. 1).

Next, the cell proliferation of bMuSCs LD increased when the chlorella extract was included at a concentration of 0.1 g/L to 10 g/L. In particular, when included at a concentration of 4 g/L, the cell proliferation increased by 139.9 % compared to the control, showing the highest increase rate. The concentration of 4 g/L was identified as Eₘₐₓ. In addition, the CC₅₀ value, which is the concentration showing 50 % of cytotoxicity, was calculated to be 23.0 g/L (FIG. 2).

Next, the cell proliferation of pMuSC LD increased within a chlorella concentration range of 0.001 g/L to 0.1 g/L and 2 g/L to 4 g/L. In particular, when included at a chlorella concentration of 0.1 g/L and 2 g/L, the cell proliferation increased by 107.5 % and 105.8 %, relatively, compared to the control. Here, Eₘₐₓ was not clear, but was confirmed to be 2 g/L. In addition, the CC₅₀ value, which is the concentration showing 50 % of cytotoxicity, was calculated to be 11.6 g/L (FIG. 3).

Next, the cell proliferation of HDF did not significantly improve at a chlorella range of 0.001 g/L to 10 g/L. Here, exact Eₘₐₓ was difficult to confirm. In addition, the CC₅₀ value, which is the concentration showing 50 % of cytotoxicity, was calculated to be 22.8 g/L (FIG. 4).

Based on the results above, it was confirmed that there was no toxicity to cells when the chlorella extract was included in the culture medium at a concentration of up to 10 g/L. Particularly, it was confirmed that the cell proliferation was remarkably excellent in bovine muscle cells when the chlorella extract was contained at a concentration of 0.8 g/L to 10 g/L.

### Example 3: Evaluation of serum replacement of chlorella extract

To evaluate the extent to which the chlorella extract can replace serum in a culture medium, the following experiment was performed.

In detail, under culture conditions in which FBS was reduced, the effect of adding the chlorella extract at the Eₘₐₓ concentration confirmed in Example 2 on the proliferation of bMuSCs was measured by a WST-8 assay. Passage 5, 6, or 15 bovine muscles cells (shank/shin, sirloin), porcine muscle cells (sirloin), and human dermal fibroblasts were seeded at a density of 5,000 cells per well in a 96-well culture plate (seeded into 3 wells per condition). After culturing the cells one day under conditions of a temperature of 37 °C and 5 % CO₂, half of the culture medium was replaced with a new culture medium containing different concentrations of FBS (0 % to 10 %) and chlorella extract (0 g/L to 4 g/L). Here, the weight ratio of the added FBS and chlorella extract is as shown in Table 1 (specific gravity of FBS was calculated as 1 g/ml).

**[Table 1]**

| **Concentration** | | **Weight per 500 mL of media (g)** | | **Weight ratio** | |
|---|---|---|---|---|---|
| **FBS (%)** | **Chlorella extract (g/L)** | **FBS** | **Chlorella extract (g/L)** | **FBS** | **Chlorella extract** |
| **0** | 0 | **0** | 0 | - | - |
| | 0.1 | | 0.05 | - | - |
| | 2 | | 1 | - | - |
| | 4 | | 2 | - | - |
| **1** | 0 | **5** | 0 | - | - |
| | 0.1 | | 0.05 | 100 | 1 |
| | 2 | | 1 | 5 | 1 |
| | 4 | | 2 | 2.5 | 1 |
| **2** | 0 | **10** | 0 | - | - |
| | 0.1 | | 0.05 | 200 | 1 |
| | 2 | | 1 | 10 | 1 |
| | 4 | | 2 | 5 | 1 |
| **5** | 0 | **25** | 0 | - | - |
| | 0.1 | | 0.05 | 500 | 1 |
| | 2 | | 1 | 25 | 1 |
| | 4 | | 2 | 12.5 | 1 |
| **10** | 0 | **50** | 0 | - | - |
| | 0.1 | | 0.05 | 1000 | 1 |
| | 2 | | 1 | 50 | 1 |
| | 4 | | 2 | 25 | 1 |

The cells were allowed to adopt to new medium while culturing the cells for one day. On the next day, the culture medium was replaced with a completely new culture medium, and on Days 0, 1, 3, and 6 since then, the WST-8 assaywas carried out to confirm the cell viability. The cell proliferation on Days 1, 3, and 6 was determined by comparing the OD450 value measured on each day with the OD450 value measured on Day 0.

As a result, when the bMuSC SM cells were treated with the chlorella extract at the Eₘₐₓ concentration, an increase in the cell proliferation was confirmed. Based on Day 6 culture, the cell proliferation rate increased by 11.9 % compared to the sample not treated with the chlorella extract in the 10 % FBS condition, and the cell proliferation increased by 33.6 % and 49.7 % in the 5% FBS condition and the 2 % FBS condition, respectively, thereby showing higher cell proliferation than the 10 % FBS control. Therefore, it can be seen that the chlorella extract can replace FBS and induce cell proliferation of the bMuSC SM cells. In detail, when the chlorella extract was included at the Eₘₐₓ of 4 g/L, the chlorella extract showed the FBS replacement rate of 80 % or more and less than 100 % (see FIG. 5 and Table 2).

**[Table 2]**

| | **Chlorella extract (g/L)** | **Cell viability (% of D0)** | | | | | |
|---|---|---|---|---|---|---|---|
| | | **Day 1 (D1)** | | **Day 3 (D3)** | | **Day 6 (D6)** | |
| | | **Average** | **standard deviation** | **Average** | **standard deviation** | **Average** | **standard deviation** |
| **0 % FBS** | **0** | 47.553 | 0.785 | 35.541 | 3.307 | 27.830 | 6.896 |
| | **2** | 45.675 | 0.869 | 39.397 | 0.817 | 29.609 | 5.266 |
| | **4** | 51.310 | 3.359 | 63.668 | 2.982 | 53.435 | 4.407 |
| **2% FBS** | **0** | 91.102 | 1.769 | 92.635 | 1.337 | 113.940 | 3.143 |
| | **2** | 90.064 | 0.617 | 116.560 | 2.682 | 145.502 | 3.670 |
| | **4** | 93.327 | 5.227 | 133.169 | 1.962 | 170.514 | 0.105 |
| **5% FBS** | **0** | 105.042 | 1.830 | 117.004 | 1.133 | 147.355 | 1.830 |
| | **2** | 120.811 | 2.215 | 137.766 | 2.154 | 168.932 | 2.412 |
| | **4** | 103.312 | 3.682 | 166.733 | 6.252 | 196.836 | 2.726 |
| **10 % FBS** | **0** | 115.571 | 1.633 | 139.298 | 2.864 | 177.163 | 4.928 |
| | **2** | 122.442 | 1.214 | 155.660 | 3.270 | 186.406 | 3.755 |
| | **4** | 113.643 | 0.856 | 171.923 | 3.591 | 198.270 | 5.065 |

Next, when the bMuSC LD cells were treated with the chlorella extract at the Eₘₐₓ concentration, an increase in the cell proliferation was confirmed. Based on Day 6 culture, the cell proliferation increased by 37.2 % and 54.0 % in the 5 % FBS condition and the 2 % FBS condition, respectively, thereby showing the cell proliferation equivalent to that of the 10 % FBS control. Therefore, it can be seen that the chlorella extract can replace FBS and induce cell proliferation of the bMuSC LD cells. In detail, when the chlorella extract was included at the Eₘₐₓ of 4 g/L, the chlorella extract showed the FBS replacement rate of 80 % or more and less than 90 % (see FIG. 6 and Table 3).

**[Table 3]**

| | **Chlorella extract (g/L)** | **Cell viability (% of D0)** | | | | | |
|---|---|---|---|---|---|---|---|
| | | **Day 1 (D1)** | | **Day 3 (D3)** | | **Day 6 (D6)** | |
| | | **Average** | **standard deviation** | **Average** | **standard deviation** | **Average** | **standard deviation** |
| **0 % FBS** | **0** | 121.518 | 11.506 | 131.370 | 9.586 | 124.915 | 20.596 |
| | **2** | 142.129 | 7.159 | 192.072 | 20.608 | 117.214 | 12.214 |
| | **4** | 145.640 | 5.849 | 206.342 | 6.982 | 131.823 | 18.128 |
| **1 % FBS** | **0** | 166.931 | 5.586 | 211.552 | 4.935 | 244.281 | 20.170 |
| | **2** | 195.583 | 14.018 | 304.530 | 7.405 | 337.712 | 3.633 |
| | **4** | 173.613 | 2.228 | 312.118 | 9.618 | 341.676 | 15.077 |
| **2 % FBS** | **0** | 182.106 | 2.410 | 231.144 | 6.231 | 260.136 | 13.838 |
| | **2** | 189.807 | 8.042 | 331.370 | 5.274 | 421.631 | 25.822 |
| | **4** | 184.258 | 6.467 | 326.387 | 19.069 | 400.680 | 9.521 |
| **5 % FBS** | **0** | 205.436 | 10.936 | 277.123 | 16.820 | 325.708 | 42.453 |
| | **2** | 236.580 | 11.232 | 344.847 | 3.265 | 420.159 | 31.917 |
| | **4** | 201.019 | 6.643 | 373.273 | 17.211 | 446.886 | 37.690 |
| **10 % FBS** | **0** | 226.614 | 13.617 | 336.127 | 15.995 | 443.828 | 47.061 |
| | **2** | 267.610 | 22.061 | 365.912 | 3.083 | 489.581 | 11.898 |
| | **4** | 263.647 | 10.466 | 390.827 | 11.869 | 462.741 | 26.345 |

Next, when the pMuSC LD was treated with the chlorella extract at a concentration of 2 g/L, an increase in the cell proliferation was confirmed. Based on Day 3 culture, the cell proliferation rate increased by 15.4 %, 49.8 %, 75.6 %, and 52.9 % in the 10 % FBS condition, 5 % FBS condition, 2 % FBS condition, and 1 % FBS condition, respectively, thereby showing the equivalent cell proliferation in the 5 % FBS condition to that of the 10 % FBS control. Therefore, it can be seen that the chlorella extract can replace FBS and induce cell proliferation of the bMuSC LD cells. In detail, when the chlorella extract was included at the Eₘₐₓ of 2 g/L, the chlorella extract showed the FBS replacement rate of 50 % or more and less than 80 % (see FIG. 7 and Table 4).

**[Table 4]**

| | **Chlorella extract (g/L)** | **Cell viability (% of D0)** | | | |
|---|---|---|---|---|---|
| | | **Day 1 (D1)** | | **Day 3 (D3)** | |
| | | **Average** | **standard deviation** | **Average** | **standard deviation** |
| **0 % FBS** | **0** | 76.021 | 3.087 | 75.626 | 7.345 |
| | **0.1** | 74.440 | 4.581 | 91.700 | 4.057 |
| | **2** | 64.822 | 9.538 | 62.055 | 15.686 |
| **1 % FBS** | **0** | 118.445 | 1.597 | 151.383 | 14.865 |
| | **0.1** | 113.702 | 2.859 | 137.945 | 3.998 |
| | **2** | 122.134 | 6.940 | 231.489 | 9.797 |
| **2 % FBS** | **0** | 127.009 | 1.425 | 184.453 | 11.805 |
| | **0.1** | 133.465 | 5.760 | 177.207 | 4.164 |
| | **2** | 120.422 | 23.873 | 323.979 | 16.836 |
| **5 % FBS** | **0** | 156.390 | 17.285 | 288.669 | 10.527 |
| | **0.1** | 129.117 | 17.967 | 305.929 | 23.617 |
| | **2** | 162.714 | 7.895 | 432.411 | 16.060 |
| **10 % FBS** | **0** | 164.032 | 10.317 | 401.581 | 17.819 |
| | **0.1** | 148.221 | 35.216 | 442.029 | 18.195 |
| | **2** | 183.531 | 8.499 | 463.505 | 29.645 |

Next, when the HDF cells were treated with the chlorella extract at a concentration of 4 g/L, there was no effect confirmed on the cell proliferation. Also, based on Day 6 culture, it was confirmed that the cell proliferation significantly reduced by the addition of the chlorella extract in all FBS concentration conditions. Therefore, unlike the case where the chlorella extract was applied to myogenic cells, there was no serum replacement effect (see FIG. 8 and Table 5).

**[Table 5]**

| | **Chlorella extract (g/L)** | **Cell viability (% of D0)** | | | | | |
|---|---|---|---|---|---|---|---|
| | | **Day 1 (D1)** | | **Day 3 (D3)** | | **Day 6 (D6)** | |
| | | **Average** | **standard deviation** | **Average** | **standard deviation** | **Average** | **standard deviation** |
| **0 % FBS** | **0** | 229.988 | 1.056 | 241.406 | 25.282 | 207.692 | 12.600 |
| | **2** | 225.481 | 18.458 | 285.457 | 11.872 | 151.262 | 9.657 |
| | **4** | 192.067 | 7.753 | 273.498 | 13.874 | 165.925 | 21.431 |
| **1 % FBS** | **0** | 247.957 | 3.525 | 303.966 | 20.012 | 307.091 | 10.407 |
| | **2** | 244.291 | 21.785 | 277.945 | 22.898 | 282.332 | 20.326 |
| | **4** | 162.921 | 3.749 | 273.978 | 39.701 | 224.700 | 31.385 |
| **2 % FBS** | **0** | 241.887 | 12.561 | 312.921 | 27.312 | 343.690 | 7.351 |
| | **2** | 241.887 | 15.562 | 290.805 | 17.415 | 316.406 | 8.500 |
| | **4** | 151.262 | 2.215 | 266.887 | 32.645 | 253.486 | 25.534 |
| **5 % FBS** | **0** | 241.106 | 16.226 | 315.685 | 23.966 | 375.962 | 7.764 |
| | **2** | 223.618 | 20.654 | 299.219 | 14.101 | 357.632 | 20.773 |
| | **4** | 149.940 | 22.288 | 280.769 | 34.651 | 268.329 | 41.234 |
| **10 % FBS** | **0** | 222.776 | 4.229 | 327.885 | 14.571 | 374.519 | 21.752 |
| | **2** | 220.793 | 9.842 | 267.788 | 11.346 | 359.255 | 10.985 |
| | **4** | 150.361 | 14.426 | 229.087 | 8.796 | 291.707 | 11.002 |

The foregoing descriptions are only for illustrating the disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

## Claims

1. A medium composition for cell culture, comprising a chlorella extract.

2. The medium composition of claim 1, wherein the chlorella extract is comprised at a concentration of 23.0 g/L or less.

3. The medium composition of claim 1, wherein the chlorella extract is comprised at a concentration of 0.4 g/L to 10 g/L.

4. The medium composition of claim 1, wherein the chlorella extract is obtained from one or more chlorellae selected from the group consisting of Chlorella vulgaris, Chlorella pyrenoidosa, Chlorella regularis, Chlorella saccharophila, Chlorella sorokiniana, and Chlorella capsulata.

5. The medium composition of claim 1, wherein the cell is a myogenic stem cell or a muscle cell.

6. The medium composition of claim 1, wherein the cell is a bovine-derived cell or a porcine-derived cell.

7. The medium composition of claim 1, further comprising serum.

8. The medium composition of claim 7, wherein the serum is one or more selected from the group consisting of fetal bovine serum (FBS), bovine calf serum (BCS), human serum, and horse serum (HS).

9. The medium composition of claim 7, wherein the serum is comprised in an amount of 0.5 parts by volume to 15 parts by volume based on 100 parts by volume of a total volume of the medium composition.

10. The medium composition of claim 7, wherein the serum is comprised at a weight ratio of 1000:1 to 1:1 relative to the chlorella extract (serum:chlorella extract).

11. A medium additive for cell culture, comprising a chlorella extract.

12. A method for culturing cells, comprising culturing separated cells by using the medium composition for cell culture of any one of claims 1 to 10.
